Europäisches Patentamt

European Patent Office

Office européen des brevets

(19)

(11) Veröffentlichungsnummer: **0 242 672 B1**

(12) # EUROPÄISCHE PATENTSCHRIFT

(45) Veröffentlichungstag der Patentschrift: **28.10.92**

(51) Int. Cl.5: **A61L 25/00**, A61L 27/00

(21) Anmeldenummer: **87104975.5**

(22) Anmeldetag: **03.04.87**

(54) **Tricalciumphosphat für Implantationsmaterialien.**

(30) Priorität: **18.04.86 DE 3613213**

(43) Veröffentlichungstag der Anmeldung:
**28.10.87 Patentblatt 87/44**

(45) Bekanntmachung des Hinweises auf die
Patenterteilung:
**28.10.92 Patentblatt 92/44**

(84) Benannte Vertragsstaaten:
**AT BE CH DE ES FR GB IT LI NL SE**

(56) Entgegenhaltungen:
EP-A- 0 016 906
EP-A- 0 131 291
EP-A- 0 202 445
FR-A- 2 370 477
GB-A- 2 032 777

(73) Patentinhaber: **MERCK PATENT GESELL-
SCHAFT MIT BESCHRÄNKTER HAFTUNG
Frankfurter Strasse 250 Postfach 4119
W-6100 Darmstadt(DE)**

(72) Erfinder: **Dingeldein, Elvira, Dr.
Am Spitzenpfad 9
W-6072 Dreieich(DE)**
Erfinder: **Wahlig, Helmut, Dr.
Roemheldweg 16
W-6100 Darmstadt(DE)**
Erfinder: **Wotschokowsky, Manfred, Dr.
Langgässerweg 52
W-6100 Darmstadt(DE)**
Erfinder: **Moddelmog, Günter
Seeheimer Strasse 49
W-6100 Darmstadt(DE)**

## Beschreibung

Die Erfindung betrifft ein resorbierbares, poröses Tricalciumphosphat, das als Zusatzstoff zu Implantationsmaterialien auf der Basis von Polyacrylaten und/oder Polymethacrylaten geeignet ist, und dessen Poren mit einem körperverträglichen, resorbierbaren Füllstoff gefüllt sind.

Es ist z.B. aus der DE-OS 29 05 878 bekannt, Implantationsmaterialien, insbesondere Knochenzementen auf Basis von Polyacrylaten und/oder Polymethacrylaten, ein im Körper resorbierbares Tricalciumphosphat zuzumischen, um eine gute knöcherne Verwachsung des Implantats mit dem umliegenden Knochengewebe zu erreichen. Ebenfalls in der DE-OS 29 05 878 wurde schon das Problem angesprochen, daß die Poren eines porösen Tricalciumphosphats durch einen geeigneten Füllstoff verschlossen werden müssen, um beim Anrühren des Zements die Aufnahme von flüssigen Acrylat- bzw. Methacrylatmonomeren in das Porensystem und dadurch verursachte Nachteile zu vermeiden. Als Füllstoffe sind dort genannt Glycerin, Wasser bzw. wäßrige Salz- oder Pufferlösungen, Ethylenglykol, niedermolekulare Polyethylenglykole und niedere Alkohole wie Ethanol, n-Propanol und Isopropanol.

Neben Tricalciumphosphat werden für solche Implantationsmaterialien weitere Zusätze vorgeschlagen bzw. auch tatsächlich benutzt, wie z.B. Röntgenkontrastmittel, Wirkstoffe, wie insbesondere Antibiotika, zur Beherrschung von Infektionen oder Fasern zur Verbesserung der mechanischen Eigenschaften des Implantats.

Obwohl jeder dieser Zusatzstoffe für sich genommen einen positiven Einfluß ausübt, besteht doch die Gefahr, daß durch die Vielzahl der Zusatzstoffe die mechanische Stabilität und das Anrührverhalten des Knochenzements negativ beeinflußt wird.

Es bestand daher die Aufgabe, einen in dieser Hinsicht verbesserten Knochenzement zu finden.

Es wurde nun gefunden, daß eine deutliche Verbesserung erzielt werden kann, wenn als Zusatzstoff ein Tricalciumphosphat eingesetzt wird, dessen Poren mit einer Mischung aus einem Antibiotikum und einem weiteren Füllstoff gefüllt sind.

Gegenstand der Erfindung ist daher ein resorbierbares, poröses Tricalciumphosphat, das als Zusatzstoff zu Implantationsmaterialien auf der Basis von Polyacrylat und/oder Polymethacrylat geeignet ist und dessen Poren mit einem körperverträglichen, resorbierbaren Füllstoff gefüllt sind, das dadurch gekennzeichnet ist, daß der Füllstoff aus zumindest zwei Bestandteilen zusammengesetzt ist, wobei ein Bestandteil ein Antibiotikum ist.

Gegenstand der Erfindung ist auch ein Verfahren zur Herstellung eines resorbierbaren, porösen Tricalciumphosphats, das als Zusatzstoff zu Implantationsmaterialien auf der Basis von Polyacrylaten und/oder Polymethacrylaten geeignet ist und dessen Poren mit einem körperverträglichen, resorbierbaren Füllstoff gefüllt sind, das dadurch gekennzeichnet ist, daß ein poröses Tricalciumphosphat mit der Lösung eines Antibiotikums und eines weiteren Füllstoffs in Berührung gebracht wird und danach das Lösungsmittel entfernt wird.

Ebenso sind Gegenstand der Erfindung ein Vorprodukt zur Herstellung eines Knochenzements und die daraus hergestellten Implantationsmaterialien, die durch einen Gehalt an dem erfindungsgemäßen Tricalciumphosphat gekennzeichnet sind.

Hauptvorteil der Erfindung ist, daß durch die verringerte Zahl der Zusatzstoffe sowohl das Anrührverhalten des Knochenzementes als auch die mechanischen Eigenschaften des ausgehärteten Implantats wesentlich verbessert werden. Weiterhin wurde überraschenderweise gefunden, daß die Freisetzung des oder der Antibiotika aus einem erfindungsgemäßen Implantat deutlich verbessert ist.

Der Begriff Tricalciumphosphat, der in der vorliegenden Anmeldung gebraucht wird, ist als Oberbegriff zu einer Anzahl von verschiedenen, im wesentlichen durch die chemische Formel $Ca_3(PO_4)_2$ zu beschreibende Materialien zu verstehen, wobei das Verhältnis Calcium : Phosphor annähernd 3 : 2 beträgt. Neben den reinen Tricalciumphosphaten, wie z.B. $\alpha$- oder $\beta$-Whitlockit sollen jedoch auch die nur annähernd durch die Formel $Ca_3(PO_4)_2$ zu beschreibenden Materialien, wie z.B. Apatite oder Phosphorit, umfaßt sein. Auf jeden Fall soll das Tricalciumphosphat im Körper resorbierbar sein.

Diese Materialien sind an sich bekannt und können nach bekannten Verfahren hergestellt werden. Im wesentlichen sind dies Fällverfahren oder Sinterverfahren oder aber eine Kombination solcher Verfahren. Fäll- oder Sinterverfahren zur Herstellung der Calciumphosphate sind in den Standardwerken der anorganischen Chemie, z.B. dem Gmelin, beschrieben. Als Ausgangsmaterialien dienen dazu in der Regel lösliche Calciumsalze und lösliche Phosphate oder aber für die Sinterverfahren z.B. CaO, $Ca(OH)_2$, $CaCO_3$ und $CaHPO_4$, die mit $P_2O_5$ oder aber untereinander zusammengesintert werden.

Besonders bevorzugt werden in der vorliegenden Erfindung durch Fällverfahren gewonnene Tricalciumphosphate eingesetzt. Diese sind in der Regel verhältnismäßig weich und weisen ein großes Porenvolumen auf, das in der Größenordnung von etwa 0,3-0,5 ml/g liegt. Die Resorption dieser durch Fällung gewonnenen Materialien ist in der Regel besser als die von gesinterten Materialien.

Erfindungsgemäß werden die Poren dieser Tricalciumphosphate mit einer Mischung eines Antibiotikums und eines weiteren Füllstoffs verschlossen. Als Antibiotika werden dazu bevorzugt Aminoglykosidantibiotika und insbesondere Gentamicin eingesetzt. Bevorzugt sind weiterhin auch Clindamycin und Lincomycin sowie auch Kombinationen von Antibiotika, wie z.B. Gentamicin mit Clindamycin.

Als weiterer Füllstoff kommen im Prinzip alle physiologisch verträglichen, vom Körper resorbierbaren und mit dem Acrylatmonomoren nicht mischbaren Stoffe in Betracht. So können z.B. die bereits in der DE-OS 29 05 878 genannten Alkohole, wie z.B. Glycerin, Ethylenglykol und niedermolekulare Polyethylenglykole, eingesetzt werden oder Zucker, wie z.B. Glukose oder Saccharose, Zuckeralkohole, wie z.B. Mannit oder Sorbit, Proteine und deren Abbauprodukte, wie z.B. Kollagen, Gelatine oder Elastin, und insbesondere auch Aminosäuren, wie z.B. Valin, Histidin, Leucin, Isoleucin, Threonin, Arginin, Lysin und Alanin. Besonders bevorzugt werden Threonin und Arginin eingesetzt.

Obwohl das Porenvolumen eines ausgewählten Tricalciumphosphats festliegt, kann doch die Menge der Füllstoffe in relativ weiten Grenzen variiert werden, ohne daß es beim Anrühren des Knochenzements zu einer Aufnahme von Monomeren in das Hohlraumsystem des Tricalciumphosphats kommt. Dies zeigt, daß offensichtlich nicht das gesamte Porenvolumen des Tricalciumphosphats mit den Füllstoffen ausgefüllt sein muß, sondern daß es offenbar ausreicht, die Poren äußerlich durch die Füllstoffe zu verschließen.

So werden bei Verwendung eines gefällten Tricalciumphosphats, das ein relativ hohes Porenvolumen besitzt, etwa 2-20, insbesondere etwa 5-15, Gew.-% Antibiotikum und etwa 0,5-5, insbesondere etwa 0,8-3,2, Gew.-% des weiteren Füllstoffs eingesetzt. Lediglich bei Verwendung eines flüssigen Füllstoffs, wie z.B. Glycerin, werden bis zu etwa 30 Gew.-% davon eingesetzt.

Diese Füllstoffe werden nach an sich bekannten Methoden auf das Tricalciumphosphat aufgebracht. So kann z.B. das Tricalciumphosphat mit einer Lösung der Füllstoffe getränkt werden und das Lösungsmittel anschließend verdampft werden. Um dabei ein intensives Eindringen der Lösung in das Porensystem des Tricalciumphosphats zu erreichen, kann dabei z.B. entweder unter Überdruck gearbeitet werden oder das Tricalciumphosphatpulver vor der Zugabe der Füllstofflösung evakuiert werden. Als Lösungsmittel für die Füllstoffe kommen sowohl organische Lösungsmittel als auch insbesondere Wasser oder wäßrige Pufferlösungen mit physiologischem pH-Wert in Frage.

Ein besonders vorteilhaftes Produkt wird durch Sprühtrocknung gewonnen. Da das vorzugsweise eingesetzte Tricalciumphosphat eine sehr kleine Teilchengröße im Bereich von etwa 2-300 μm,

insbesondere etwa 20-200 μm, besitzt, können Suspensionen des Tricalciumphosphats in einer Lösung der Füllstoffe nach üblichen Methoden sprühgetrocknet werden. Die dabei anzuwendenden Bedingungen, wie Konzentration der Suspension, Temperatur und Druck sind dem Fachmann geläufig und können notfalls in Anlehnung an in Lehrbüchern, wie z.B. in Sucker, Fuchs und Speiser "Pharmazeutische Technologie", Georg-Thieme-Verlag, Stuttgart, 1978, beschriebenen Methoden durch wenige orientierende Versuche in bezug auf die jeweils verwendeten Komponenten optimiert werden.

Die nach einer dieser Methoden als frei fließendes Pulver erhaltenen Tricalciumphosphatteilchen können unmittelbar bzw. nach Sterilisierung, z.B. durch Bestrahlung oder durch Ethylenoxidbegasung, bei der Herstellung von Knochenzementen eingesetzt werden. Die bekannten Knochenzemente werden so zubereitet, daß etwa zwei Teile eines feinteiligen, einen Polymerisationskatalysator (z.B. Dibenzoylperoxid) enthaltenden Präpolymerisats, insbesondere Polymethylmethacrylat oder eines Mischpolymerisats aus Methylacrylat und Methylmethacrylat, mit etwa einem Teil des flüssigen Monomeren, z.B. Acrylsäure oder Methacrylsäuremethylester oder deren Gemische, das einen Beschleuniger (z.B. Dimethyl-p-toluidin) enthält, zu einer formbaren Masse gemischt werden, die in den Körper implantiert wird und dort aushärtet. Solche Knochenzemente sind z.B. unter dem Warenzeichen Palacos® im Handel.

Knochenzemente mit dem erfindungsgemäßen Tricalciumphosphat werden in analoger Weise so hergestellt, daß entweder eine Mischung der drei Bestandteile Präpolymerisat, Tricalciumphosphat und Monomer erfolgt oder daß Tricalciumphosphat vorab einem der beiden anderen Bestandteile zugemischt wird.

Vorzugsweise wird man den Knochenzement so in den Handel bringen, daß die festen und flüssigen Bestandteile zwar getrennt, aber in aufeinander abgestimmten Mengen in einer gebrauchsfertigen Packung vorliegen. Dabei wird vorzugsweise ein Verhältnis von etwa 40 g Feststoff zu 20 ml flüssigen Monomeren eingehalten. Der Anteil des erfindungsgemäßen Tricalciumphosphats an der Feststoffkomponente liegt dabei in der Regel bei etwa 5-30, insbesondere etwa 8-20, Gew.-%.

Neben dem erfindungsgemäßen Tricalciumphosphat können der Feststoffkomponente weitere Zusätze zugemischt werden. So kann z.B. in Mengen von 0-20 Gew.-% ein weiterer Tricalciumphosphatanteil ohne Füllstoffe, wie z.B. ein im wesentlichen porenfreier, gesinterter Apatit, oder auch ein Röntgenkontrastmittel, wie z.B. Zirkondioxid, zugemischt werden. Auf letzteren Bestandteil kann jedoch verzichtet werden, da schon die Tricalciumphosphatkomponenten einen ausreichenden Röntgenkontrast bewirken. Zur Verbesserung der mechanischen Eigenschaften des Implantats können auch in Mengen von etwa 0-30 Gew.-% Faserbestandteile, wie z.B. Kohlenstoff- oder Glasfasern oder Kunststoffasern, wie z.B. solche aus Polymethylmethacrylat oder Aramit, beigefügt werden. Zusätzlich können auch andere Materialien, wie insbesondere bioaktive Glaskeramiken, in Mengen von 0-50 Gew.-% enthalten sein. In jedem Fall wird die Feststoffkomponente jedoch so bemessen, daß das Verhältnis von 40 g Feststoff zu 20 ml Monomer im wesentlichen eingehalten wird, d.h. mit zunehmendem Anteil an anderen Zusätzen wird der Anteil des Präpolymeren verringert.

Es wurde bereits erwähnt, daß, verglichen mit einem Implantationsmaterial, dem ein pulverförmiges Antibiotikum zugemischt wurde, die Freisetzung des Antibiotikums aus einem erfindungsgemäßen Implantationsmaterial deutlich verbessert ist. Tatsächlich findet man eine Freisetzung, die um den Faktor 5-10 verbessert ist. Damit steht eine sehr wertvolle und vorteilhafte neue Tricalciumphosphatkomponente für die Herstellung von Knochenzementen zur Verfügung.

### Beispiel 1

In einer auf pH 7,4 eingestellten Lösung von 0,8 g L-Arginin und 1,6 g Gentamicinsulfat werden 97,6 g Tricalciumphosphat (Fa. E. Merck, Darmstadt, Art.-Nr. 2194) einer Teilchengröße unterhalb 63 $\mu$m suspendiert und die Suspension wird in einer Sprühtrockenanlage versprüht (Düsendruck: 2 bar; Luftmenge 380 m$^3$/h; Temperatur-Eingang: 200 ° C; Temperatur-Ausgang: 65 ° C).

### Beispiel 2

Es wird analog Beispiel 1 gearbeitet, wobei jedoch 1,6 g L-Arginin und 96,8 g Tricalciumphosphat eingesetzt werden.

### Beispiel 3

Es wird analog Beispiel 1 gearbeitet, wobei jedoch 1,6 g L-Arginin, 4,0 g Gentamicin und 94,4 g Tricalciumphosphat eingesetzt werden.

### Beispiel 4

Es wird analog Beispiel 1 gearbeitet, wobei jedoch 1,6 g L-Arginin, 8,0 g Gentamicin und 90,4 g Tricalciumphosphat eingesetzt werden.

### Beispiel 5

Es wird analog Beispiel 1 gearbeitet, wobei jedoch 1,6 g L-Arginin, 16,0 g Gentamicin und 82,4 g Tricalciumphosphat eingesetzt werden.

### Beispiel 6

Es wird analog Beispiel 1 gearbeitet, wobei jedoch 5,0 g L-Arginin, 8,0 g Gentamicin und 87 g Tricalciumphosphat eingesetzt werden.

### Beispiele 7-12

Es wird analog den Beispielen 1-6 gearbeitet, wobei jedoch anstelle von L-Arginin L-Threonin eingesetzt wird.

### Patentansprüche

1. Resorbierbares, poröses Tricalciumphosphat, das als Zusatzstoff zu Implantationsmaterialien auf der Basis von Polyacrylaten und/oder Polymethacrylaten geeignet ist, und dessen Poren mit einem körperverträglichen, resorbierbaren Füllstoff gefüllt sind, dadurch gekennzeichnet, daß der Füllstoff aus zumindest zwei Bestandteilen zusammengesetzt ist, wobei ein Bestandteil ein Antibiotikum ist.

2. Tricalciumphosphat nach Anspruch 1, dadurch gekennzeichnet, daß neben dem Antibiotikum eine Aminosäure enthalten ist.

3. Tricalciumphosphat nach Anspruch 1 oder 2, dadurch gekennzeichnet, daß als Antibiotikum ein Aminoglykosidantibiotikum enthalten ist.

4. Verfahren zur Herstellung eines resorbierbaren, porösen Tricalciumphosphats, das als Zusatzstoff zu Implantationsmaterialien auf der Basis von Polyacrylat und/oder Polymethacrylat geeignet ist, und dessen Poren mit einem körperverträglichen, resorbierbaren Füllstoff gefüllt sind, dadurch gekennzeichnet, daß ein poröses Tricalciumphosphat mit der Lösung eines Antibiotikums und eines weiteren Füllstoffs in Berührung gebracht und danach das Lösungsmittel entfernt wird.

5. Verfahren nach Anspruch 4, dadurch gekennzeichnet, daß das Tricalciumphosphat in der Lösung eines Antibiotikums und eines weiteren Füllstoffs suspendiert und die Suspension dann sprühgetrocknet wird.

6. Verfahren nach Anspruch 4 oder 5, dadurch gekennzeichnet, daß als Antibiotikum ein Aminoglykosidantibiotikum und als weiterer Füllstoff eine Aminosäure eingesetzt wird.

7. Vorprodukt zur Herstellung eines Knochenzements, das in einer gebrauchsfertigen Pak-
kungseinheit eine pulverförmige Feststoffkomponente auf der Basis von Polyacrylat
und/oder Polymethacrylat sowie eine flüssige
Monomerkomponente enthält, dadurch gekennzeichnet, daß die Feststoffkomponente 5-30
Gew.-% Tricalciumphosphat nach Anspruch 1
enthält.

8. Implantationsmaterialien, insbesondere Knochenzemente auf Basis von Polyacrylaten
und/oder Polymethacrylaten, dadurch gekennzeichnet, daß sie 3-20 Gew.-% eines Tricalciumphosphats nach Anspruch 1 enthalten.

## Claims

1. Absorbable porous tricalcium phosphate which
is suitable as an additive to implant materials
based on polyacrylates and/or polymethacrylates, its pores being filled with an
absorbable filler tolerated by the body, characterized in that the filler is composed of at
least two constituents, one constituent being
an antibiotic.

2. Tricalcium phosphate according to Claim 1,
characterized in that it contains an aminoacid,
in addition to the antibiotic.

3. Tricalcium phosphate according to Claim 1 or
2, characterized in that it contains an
aminoglycoside antibiotic as the antibiotic.

4. Process for the preparation of an absorbable
porous tricalcium phosphate which is suitable
as an additive for implant materials based on
polyacrylate and/or polymethacrylate, its pores
being filled with an absorbable filler which is
tolerated by the body, characterized in that a
porous tricalcium phosphate is brought into
contact with the solution of an antibiotic and
another filler and the solvent is then removed.

5. Process according to Claim 4, characterized in
that the tricalcium phosphate is suspended in
the solution of an antibiotic and another filler
and the suspension is then spray-dried.

6. Process according to Claim 4 or 5, characterized in that an aminoglycoside antibiotic is
used as the antibiotic and an aminoacid is
used as the other filler.

7. Precursor for the preparation of a cone cement
which contains a pulverulent solid component
based on polyacrylate and/or polymethacrylate and a liquid monomer component in a ready-
to-use pack unit, characterized in that the solid
component contains 5-30% by weight of tricalcium phosphate according to Claim 1.

8. Implant materials, in particular bone cements
based on polyacrylates and/or polymethacrylates, characterized in that they contain 3-20% by weight of a tricalcium phosphate
according to Claim 1.

## Revendications

1. Phosphate tricalcique poreux résorbable
convenant comme additif pour les matériaux
pour implants à base de polyacrylate et/ou de
polyméthacrylate et dont les pores sont remplis d'une matière de charge résorbable et
physiologiquement compatible , caractérisé en
ce que la matière de charge est formée d'au
moins deux constituants, l'un de ces constituants étant un antibiotique.

2. Phosphate tricalcique selon la revendication 1,
caractérisé en ce qu'il contient un acide aminé
outre l'antibiotique.

3. Phosphate tricalcique selon la revendication 1
ou 2, caractérisé en ce qu'il contient comme
antibiotique un antibiotique à base d'aminoglycosides.

4. Procédé de préparation d'un phosphate tricalcique poreux résorbable qui convient comme
additif pour les matériaux pour implants à base
de polyacrylate et/ou de polyméthacrylate et
dont les pores sont remplis d'une manière de
charge résorbable physiologiquement comptible, caractérisé en ce que l'on met un phosphate tricalcique poreux en contact avec une
solution d'un antibiotique et d'une autre matière de charge et que le solvant est ensuite
éliminé.

5. Procédé selon la revendication 4, caractérisé
en ce que le phosphate tricalcique est mis en
suspension dans la solution d'un antibiotique
d'une autre matière de charge et que la suspension est ensuite séchée par pulvérisation.

6. Procédé selon la revendication 4 ou 5, caractérisé en ce que l'on utilise comme antibiotique
un antibiotique à base d'aminoglycosides et,
comme autre matière de charge, un acide aminé.

7. Produit intermédiaire pour la préparation d'un
ciment pour os qui contient, dans une unité d'emballage prête à l'emploi, un constituant solide pulvérulent à base de polyacrylate et/ou de polyméthacrylate ainsi qu'un composant monomère liquide, caractérisé en ce que le composant solide contient de 5 à 30% en poids de phosphate tricalcique selon la revendication 1.

8. Matériaux pour implants, et en particulier ciments pour os à base de polyacrylate et /ou de polyméthacrylate , caractérisés en ce qu'ils contiennent de 3 à 20% en poids d'un phosphate tricalcique selon la revendication 1.